# EUROPEAN PATENT APPLICATION

(11) **EP 4 485 328 A1**
(43) Date of publication of application: **01.01.2025**
(21) Application number: 22932377.9
(22) Date of filing: 19.04.2022
(51) Int. Cl.: G06Q 30/06, G06Q 10/08, G06V 10/10, G06N 20/00, G06N 3/08, G16H 10/60, G16H 20/60, G06Q 10/087, G06Q 30/0601

(54) **ARTIFICIAL INTELLIGENCE DEVICE FOR RECOMMENDING PRODUCT ON BASIS OF PRODUCT STOCK WITHIN REFRIGERATOR AND METHOD THEREFOR**

(30) Priority: 16.03.2022 KR 20220032603
(71) Applicant: LG Electronics Inc., Yeongdeungpo-gu Seoul 07336 (KR)
(72) Inventor: CHOI, Shinhye, Seoul 06772 (KR); KIM, Joongrock, Seoul 06772 (KR); LEE, Yongsuk, Seoul 06772 (KR); KIM, Suyeon, Seoul 06772 (KR); KIM, Kokeun, Seoul 06772 (KR); CHUNG, Woocheol, Seoul 06772 (KR); LEE, Kamin, Seoul 06772 (KR); KAM, Mingyoung, Seoul 06772 (KR)
(74) Representative: Frenkel, Matthias Alexander
(86) International application number: PCT/KR2022/005589
(87) International publication number: WO 2023/177010

(57) **Abstract**

An artificial intelligence according to one embodiment of a present disclosure comprises a communicator configured to receive a product image in which at least one product is captured in a refrigerator, and a processor configured to obtain at least one product information based on the product image, generate a stock list for products stored in the refrigerator based on the product information, determine a user-preferred product based on a change in the stock quantity of each of at least one product included in the stock list, and determine whether the user-preferred product is a subscription available product and recommend the user-preferred product as a subscription product if the user-preferred product is determined as subscription available product.

## Description

### [Technical Field]

The present disclosure relates to an artificial intelligence device that recommends products based on product stock in a refrigerator and an operating method thereof.

### [Background Art]

Artificial intelligence is a field of computer engineering and information technology that studies how computers can think, learn, and develop themselves, which are things that humans can do. It means that computers can imitate human intelligent behavior.

In addition, artificial intelligence does not exist by itself, but is directly or indirectly related to other fields of computer science. In particular, in modern times, attempts are being made to introduce artificial intelligence elements into various fields of information technology and utilize them to solve problems in those fields.

Meanwhile, technologies that use artificial intelligence to recognize and learn the surrounding situation and provide the information that the user wants in the desired form or perform the actions or functions that the user wants are being actively studied.

And electronic devices that provide these various operations and functions can be called artificial intelligence devices.

Meanwhile, users store products that they frequently consume in the refrigerator.

However, it is difficult to always remember how many of the products that they prefer are stored in the refrigerator.

In addition, there are many cases where users realize too late that their desired products are out of stock in the refrigerator.

In addition, users may not know how the products they consume affect their health.

Therefore, there is an increasing need for technology that can use artificial intelligence technology to store products that users prefer in the refrigerator and recommend products suitable for their health.

### [Disclosure]

### [Technical Problem]

The purpose of this present disclosure is to solve the above-mentioned problems and other problems.

The purpose of this present disclosure is to provide an artificial intelligence device and method thereof that recommends products based on product stock in a refrigerator.

The purpose of this present disclosure is to provide an artificial intelligence device and method thereof that automatically recognizes products stored in a refrigerator and generates a stock list of products in the refrigerator to automatically determine products preferred by a user.

The purpose of this present disclosure is to provide an artificial intelligence device and method thereof that sets a product preferred by a user as a subscription product so that an order is automatically received according to changes in stock in the refrigerator, thereby maintaining the stock of the product preferred by the user constant.

The purpose of this present disclosure is to provide an artificial intelligence device and method thereof that can recommend a product that is healthier than the product withdrawn by the user by considering the user's health information.

### [Technical Solution]

One embodiment of the present disclosure provides an artificial intelligence device including a communicator configured to receive a product image in which at least one product is captured in a refrigerator, and a processor configured to obtain at least one product information based on the product image, generate a stock list for products stored in the refrigerator based on the product information, determine a user-preferred product based on a change in the stock quantity of each of at least one product included in the stock list, and determine whether the user-preferred product is a subscription available product and recommend the user-preferred product as a subscription product if the user-preferred product is determined as subscription available product.

### [Advantageous Effects]

According to an embodiment of the present disclosure, a product can be recommended based on a product stock in a refrigerator.

In addition, according to various embodiments of the present disclosure, a product stored in the refrigerator can be automatically recognized to generate a stock list of products in the refrigerator, and a product preferred by the user can be automatically determined.

In addition, according to various embodiments of the present disclosure, a product preferred by the user can be set as a subscription product so that an order is automatically made according to a change in the stock in the refrigerator, thereby maintaining the stock of the user-preferred product constant.

In addition, according to various embodiments of the present disclosure, a product that is healthier than the product withdrawn by the user can be recommended by considering the user's health information.

### [Description of Drawings]

FIG. 1 illustrates an AI device according to an embodiment of the present disclosure.
FIG. 2 illustrates an AI server according to an embodiment of the present disclosure.
FIG. 3 illustrates an AI system according to an embodiment of the present disclosure.
FIG. 4 illustrates an AI device 100 according to one another embodiment of the present disclosure.
FIG. 5 is a flowchart for explaining a product recommendation method according to an embodiment of the present disclosure.
FIG. 6 is a drawing for explaining a refrigerator that captures a product according to an embodiment of the present disclosure.
FIG. 7 is a drawing for explaining a product recognition model according to an embodiment of the present disclosure.
FIG. 8 is a drawing for explaining a product image in which product information is recognized according to one embodiment of the present disclosure.
FIG. 9 is a drawing showing a delivery order alarm screen according to one embodiment of the present disclosure.
FIG. 10 is a flowchart for explaining a product recommendation method based on user health information according to one embodiment of the present disclosure.

### [Best Mode]

Hereinafter, embodiments are described in more detail with reference to accompanying drawings and regardless of the drawings symbols, same or similar components are assigned with the same reference numerals and thus repetitive for those are omitted. Since the suffixes "module" and "unit" for components used in the following description are given and interchanged for easiness in making the present disclosure, they do not have distinct meanings or functions. In the following description, detailed descriptions of well-known functions or constructions will be omitted because they would obscure the inventive concept in unnecessary detail. Also, the accompanying drawings are used to help easily understanding embodiments disclosed herein but the technical idea of the inventive concept is not limited thereto. It should be understood that all of variations, equivalents or substitutes contained in the concept and technical scope of the present disclosure are also included.

Although the terms including an ordinal number, such as "first" and "second", are used to describe various components, the components are not limited to the terms. The terms are used to distinguish between one component and another component.

It will be understood that when a component is referred to as being coupled with/to" or "connected to" another component, the component may be directly coupled with/to or connected to the another component or an intervening component may be present therebetween. Meanwhile, it will be understood that when a component is referred to as being directly coupled with/to" or "connected to" another component, an intervening component may be absent therebetween.

### <AI: Artificial Intelligence>

AI refers to a field that studies artificial intelligence or the methodologies for creating it, and machine learning refers to a field that defines various problems in the field of artificial intelligence and studies the methodologies for solving them. Machine learning is also defined as an algorithm that improves the performance of a task through constant experience with that task.

An artificial neural network (ANN) is a model used in machine learning, and can refer to a model with problem-solving capabilities that is composed of artificial neurons (nodes) that form a network by combining synapses. An artificial neural network can be defined by the connection pattern between neurons in different layers, the learning process that updates model parameters, and the activation function that generates output values.

An artificial neural network may include an input layer, an output layer, and optionally one or more hidden layers. Each layer may include one or more neurons, and the artificial neural network may include synapses connecting neurons. In an artificial neural network, each neuron may output a function value of an activation function for input signals, weights, and biases input through synapses.

Model parameters refer to parameters determined through learning, including the weights of synaptic connections and the biases of neurons. Hyperparameters refer to parameters that must be set before learning in machine learning algorithms, including learning rate, number of repetitions, mini-batch size, and initialization functions.

The purpose of learning an artificial neural network can be seen as determining model parameters that minimize a loss function. The loss function can be used as an indicator to determine optimal model parameters during the learning process of an artificial neural network.

Machine learning can be classified into supervised learning, unsupervised learning, and reinforcement learning depending on the learning method.

Supervised learning refers to a method of training an artificial neural network when labels for training data are given, and the labels can refer to the correct answer (or result value) that the artificial neural network must infer when training data is input to the artificial neural network. Unsupervised learning can refer to a method of training an artificial neural network when labels for training data are not given. Reinforcement learning can refer to a learning method that trains an agent defined in a certain environment to select an action or action sequence that maximizes the cumulative reward in each state.

Machine learning implemented with a deep neural network (DNN) that includes multiple hidden layers among artificial neural networks is also called Deep Learning, and deep learning is a part of machine learning. Hereinafter, machine learning is used to mean including deep learning.

Object detection models using machine learning include the YOLO (You Only Look Once) model in single-step method and Faster R-CNN (Regions with Convolution Neural Networks) model in two-step method.

The YOLO (You Only Look Once) model is a model that can predict objects and the locations of those objects within an image by looking at the image only once.

The YOLO (You Only Look Once) model divides the original image into grids of the same size. Then, for each grid, the number of bounding boxes specified in a predefined form centered on the center of the grid is predicted, and the confidence is calculated based on this.

After that, whether the image contains an object or just a background is included, and the location with high object confidence is selected so that the object category can be identified.

The Faster R-CNN (Regions with Convolution Neural Networks) model is a model that can detect objects faster than the RCNN model and the Fast RCNN model.

The Faster R-CNN (Regions with Convolution Neural Networks) model is explained in detail.

First, a feature map is extracted from the image using a CNN (Convolution Neural Network) model. Based on the extracted feature map, multiple Regions of Interest (RoI) are extracted. RoI pooling is performed for each region of interest.

RoI pooling is a process of setting a grid to a predetermined size of H x W for the feature map onto which the region of interest is projected, extracting the largest value for each cell included in each grid, and extracting a feature map having the size of H x W.

Feature vectors are extracted from feature maps having the size of H x W, and object identification information can be obtained from the feature vectors.

### < Robot>

A robot can refer to a machine that automatically processes or operates a given task by its own ability. In particular, a robot that has the ability to recognize the environment, make judgments on its own, and perform actions can be called an intelligent robot.

Robots can be classified into industrial, medical, household, and military types depending on their purpose or field of use.

A robot can perform various physical actions, such as moving robot joints, by having a driving unit that includes an actuator or motor. In addition, a mobile robot can have a driving unit that includes wheels, brakes, and propellers, and can drive on the ground or fly in the air through the driving unit.

### <Self-Driving>

Autonomous driving refers to technology that drives itself, and an autonomous vehicle refers to a vehicle that drives without user intervention or with minimal user intervention.

For example, autonomous driving can include technology that maintains the driving lane, technology that automatically adjusts speed such as adaptive cruise control, technology that automatically drives along a set path, and technology that automatically sets a path and drives when a destination is set.

A vehicle includes all vehicles equipped with only an internal combustion engine, hybrid vehicles equipped with both an internal combustion engine and an electric motor, and electric vehicles equipped with only an electric motor, and can include not only cars but also trains, motorcycles, etc.

In this case, an autonomous vehicle can be viewed as a robot with autonomous driving functions.

### <XR: eXtended Reality>

Extended reality is a general term for virtual reality (VR), augmented reality (AR), and mixed reality (MR). VR technology provides objects or backgrounds in the real world as CG images only, AR technology provides virtual CG images on top of images of real objects, and MR technology is a computer graphics technology that mixes and combines virtual objects in the real world.

MR technology is similar to AR technology in that it shows real objects and virtual objects together. However, there is a difference in that while AR technology uses virtual objects to complement real objects, MR technology uses virtual objects and real objects as equals.

XR technology can be applied to HMD (Head-Mount Display), HUD (Head-Up Display), mobile phones, tablet PCs, laptops, desktops, TVs, digital signage, etc., and devices to which XR technology is applied can be called XR devices.

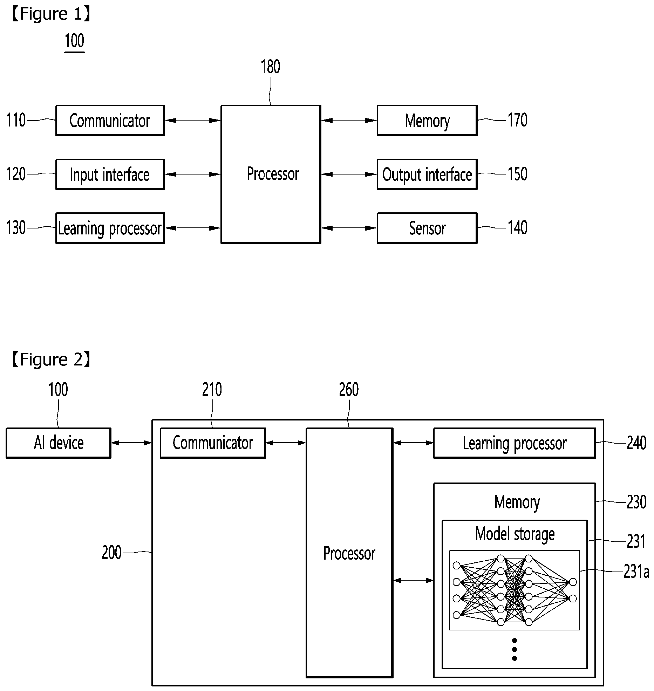

Fig. 1 illustrates an AI device 100 according to an embodiment of the present disclosure.

The AI device 100 may be implemented by a stationary device or a mobile device, such as a TV, a projector, a mobile phone, a smartphone, a desktop computer, a notebook, a digital broadcasting terminal, a personal digital assistant (PDA), a portable multimedia player (PMP), a navigation device, a tablet PC, a wearable device, a set-top box (STB), a DMB receiver, a radio, a washing machine, a refrigerator, a desktop computer, a digital signage, a robot, a vehicle, and the like.

Referring to Fig. 1, the AI device 100 may include a communicator 110, an input interface 120, a learning processor 130, a sensor 140, an output interface 150, a memory 170, and a processor 180.

The communicator 110 may transmit and receive data to and from external devices such as other AI devices 100a to 100e and the AI server 200 by using wire/wireless communication technology. For example, the communicator 110 may transmit and receive sensor information, a user input, a learning model, and a control signal to and from external devices.

The communication technology used by the communicator 110 includes GSM (Global System for Mobile communication), CDMA (Code Division Multi Access), LTE (Long Term Evolution), 5G, WLAN (Wireless LAN), Wi-Fi (Wireless-Fidelity), Bluetooth^{™}, RFID (Radio Frequency Identification), Infrared Data Association (IrDA), ZigBee, NFC (Near Field Communication), and the like.

The input interface 120 may acquire various kinds of data.

At this time, the input interface 120 may include a camera for inputting a video signal, a microphone for receiving an audio signal, and a user input interface for receiving information from a user. The camera or the microphone may be treated as a sensor, and the signal acquired from the camera or the microphone may be referred to as sensing data or sensor information.

The input interface 120 may acquire a learning data for model learning and an input data to be used when an output is acquired by using learning model. The input interface 120 may acquire raw input data. In this case, the processor 180 or the learning processor 130 may extract an input feature by preprocessing the input data.

The learning processor 130 may learn a model composed of an artificial neural network by using learning data. The learned artificial neural network may be referred to as a learning model. The learning model may be used to an infer result value for new input data rather than learning data, and the inferred value may be used as a basis for determination to perform a certain operation.

At this time, the learning processor 130 may perform AI processing together with the learning processor 240 of the AI server 200.

At this time, the learning processor 130 may include a memory integrated or implemented in the AI device 100. Alternatively, the learning processor 130 may be implemented by using the memory 170, an external memory directly connected to the AI device 100, or a memory held in an external device.

The sensor 140 may acquire at least one of internal information about the AI device 100, ambient environment information about the AI device 100, and user information by using various sensors.

Examples of the sensors included in the sensor 140 may include a proximity sensor, an illuminance sensor, an acceleration sensor, a magnetic sensor, a gyro sensor, an inertial sensor, an RGB sensor, an IR sensor, a fingerprint recognition sensor, an ultrasonic sensor, an optical sensor, a microphone, a lidar, and a radar.

The output interface 150 may generate an output related to a visual sense, an auditory sense, or a haptic sense.

At this time, the output interface 150 may include a display for outputting visual information, a speaker for outputting auditory information, and a haptic module for outputting tactile information.

The memory 170 may store data that supports various functions of the AI device 100. For example, the memory 170 may store input data acquired by the input interface 120, learning data, a learning model, a learning history, and the like.

The processor 180 may determine at least one executable operation of the AI device 100 based on information determined or generated by using a data analysis algorithm or a machine learning algorithm. And, the processor 180 may control the components of the AI device 100 to execute the determined operation.

To this end, the processor 180 may request, search, receive, or utilize data of the learning processor 130 or the memory 170. The processor 180 may control the components of the AI device 100 to execute the predicted operation or the operation determined to be desirable among the at least one executable operation.

When the connection of an external device is required to perform the determined operation, the processor 180 may generate a control signal for controlling the external device and may transmit the generated control signal to the external device.

The processor 180 may acquire intention information for the user input and may determine the user's requirements based on the acquired intention information.

The processor 180 may acquire the intention information corresponding to the user input by using at least one of a speech to text (STT) engine for converting speech input into a text string or a natural language processing (NLP) engine for acquiring intention information of a natural language.

At least one of the STT engine or the NLP engine may be configured as an artificial neural network, at least part of which is learned according to the machine learning algorithm. At least one of the STT engine or the NLP engine may be learned by the learning processor 130, may be learned by the learning processor 240 of the AI server 200, or may be learned by their distributed processing.

The processor 180 may collect history information including the operation contents of the AI apparatus 100 or the user's feedback on the operation and may store the collected history information in the memory 170 or the learning processor 130 or transmit the collected history information to the external device such as the AI server 200. The collected history information may be used to update the learning model.

The processor 180 may control at least part of the components of AI device 100 so as to drive an application program stored in memory 170. Furthermore, the processor 180 may operate two or more of the components included in the AI device 100 in combination so as to drive the application program.

Fig. 2 illustrates an AI server 200 according to an embodiment of the present disclosure.

Referring to Fig. 2, the AI server 200 may refer to a device that learns an artificial neural network by using a machine learning algorithm or uses a learned artificial neural network. Here, the AI server 200 may include a plurality of servers to perform distributed processing, and may be defined as a 5G network. At this time, the AI server 200 may be included as a partial configuration of the AI device 100, and may perform at least part of the AI processing together.

The AI server 200 may include a communicator 210, a memory 230, a learning processor 240, a processor 260, and the like.

The communicator 210 can transmit and receive data to and from an external device such as the AI device 100.

The memory 230 may include a model storage 231. The model storage 231 may store a learning or learned model (or an artificial neural network 231a) through the learning processor 240.

The learning processor 240 may learn the artificial neural network 231a by using the learning data. The learning model may be used in a state of being mounted on the AI server 200 of the artificial neural network, or may be used in a state of being mounted on an external device such as the AI device 100.

The learning model may be implemented in hardware, software, or a combination of hardware and software. If all or part of the learning models are implemented in software, one or more instructions that constitute the learning model may be stored in memory 230.

The processor 260 may infer the result value for new input data by using the learning model and may generate a response or a control command based on the inferred result value.

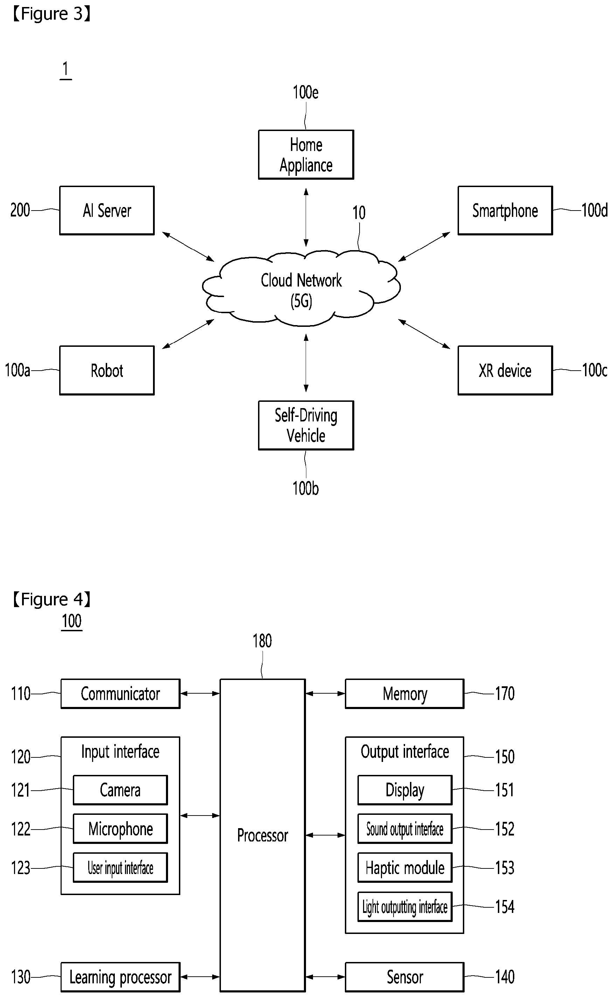

Fig. 3 illustrates an AI system 1 according to an embodiment of the present disclosure.

Referring to Fig. 3, in the AI system 1, at least one of an AI server 200, a robot 100a, a self-driving vehicle 100b, an XR device 100c, a smartphone 100d, or a home appliance 100e is connected to a cloud network 10. The robot 100a, the self-driving vehicle 100b, the XR device 100c, the smartphone 100d, or the home appliance 100e, to which the AI technology is applied, may be referred to as AI devices 100a to 100e.

The cloud network 10 may refer to a network that forms part of a cloud computing infrastructure or exists in a cloud computing infrastructure. The cloud network 10 may be configured by using a 3G network, a 4G or LTE network, or a 5G network.

That is, the devices 100a to 100e and 200 configuring the AI system 1 may be connected to each other through the cloud network 10. In particular, each of the devices 100a to 100e and 200 may communicate with each other through a base station, but may directly communicate with each other without using a base station.

The AI server 200 may include a server that performs AI processing and a server that performs operations on big data.

The AI server 200 may be connected to at least one of the AI devices constituting the AI system 1, that is, the robot 100a, the self-driving vehicle 100b, the XR device 100c, the smartphone 100d, or the home appliance 100e through the cloud network 10, and may assist at least part of AI processing of the connected AI devices 100a to 100e.

At this time, the AI server 200 may learn the artificial neural network according to the machine learning algorithm instead of the AI devices 100a to 100e, and may directly store the learning model or transmit the learning model to the AI devices 100a to 100e.

At this time, the AI server 200 may receive input data from the AI devices 100a to 100e, may infer the result value for the received input data by using the learning model, may generate a response or a control command based on the inferred result value, and may transmit the response or the control command to the AI devices 100a to 100e.

Alternatively, the AI devices 100a to 100e may infer the result value for the input data by directly using the learning model, and may generate the response or the control command based on the inference result.

Hereinafter, various embodiments of the AI devices 100a to 100e to which the above-described technology is applied will be described. The AI devices 100a to 100e illustrated in Fig. 3 may be regarded as a specific embodiment of the AI device 100 illustrated in Fig. 1.

### <AI+robot>

The robot 100a, to which the AI technology is applied, may be implemented as a guide robot, a carrying robot, a cleaning robot, a wearable robot, an entertainment robot, a pet robot, an unmanned flying robot, or the like.

The robot 100a may include a robot control module for controlling the operation, and the robot control module may refer to a software module or a chip implementing the software module by hardware.

The robot 100a may acquire state information about the robot 100a by using sensor information acquired from various kinds of sensors, may detect (recognize) surrounding environment and objects, may generate map data, may determine the route and the travel plan, may determine the response to user interaction, or may determine the operation.

Here, the robot 100a may use the sensor information acquired from at least one sensor among the lidar, the radar, and the camera so as to determine the travel route and the travel plan.

The robot 100a may perform the above-described operations by using the learning model composed of at least one artificial neural network. For example, the robot 100a may recognize the surrounding environment and the objects by using the learning model, and may determine the operation by using the recognized surrounding information or object information. The learning model may be learned directly from the robot 100a or may be learned from an external device such as the AI server 200.

At this time, the robot 100a may perform the operation by generating the result by directly using the learning model, but the sensor information may be transmitted to the external device such as the AI server 200 and the generated result may be received to perform the operation.

The robot 100a may use at least one of the map data, the object information detected from the sensor information, or the object information acquired from the external apparatus to determine the travel route and the travel plan, and may control the driving unit such that the robot 100a travels along the determined travel route and travel plan.

The map data may include object identification information about various objects arranged in the space in which the robot 100a moves. For example, the map data may include object identification information about fixed objects such as walls and doors and movable objects such as pot and desks. The object identification information may include a name, a type, a distance, and a position.

In addition, the robot 100a may perform the operation or travel by controlling the driving unit based on the control/interaction of the user. At this time, the robot 100a may acquire the intention information of the interaction due to the user's operation or speech utterance, and may determine the response based on the acquired intention information, and may perform the operation.

### <AI+ Autonomous driving>

The autonomous vehicle 100b can be implemented as a mobile robot, vehicle, unmanned aerial vehicle, etc. by applying AI technology.

The autonomous vehicle 100b can include an autonomous driving control module for controlling the autonomous driving function, and the autonomous driving control module can mean a software module or a chip that implements it as hardware. The autonomous driving control module can be included internally as a component of the autonomous vehicle 100b, but can also be configured as a separate hardware and connected to the outside of the autonomous vehicle 100b.

The autonomous vehicle 100b can obtain status information of the autonomous vehicle 100b, detect (recognize) the surrounding environment and objects, generate map data, determine a travel path and driving plan, or determine an action by using sensor information obtained from various types of sensors.

Here, the autonomous vehicle 100b may use sensor information acquired from at least one sensor among lidar, radar, and camera, similar to the robot 100a, to determine a movement path and driving plan.

In particular, the autonomous vehicle 100b may recognize an environment or object in an area where the field of view is obscured or an area over a certain distance by receiving sensor information from external devices, or may receive information recognized directly from external devices.

The autonomous vehicle 100b may perform the above-described operations using a learning model composed of at least one artificial neural network. For example, the autonomous vehicle 100b may recognize the surrounding environment and objects using the learning model, and may determine a driving route using the recognized surrounding environment information or object information. Here, the learning model may be learned directly by the autonomous vehicle 100b or may be learned from an external device such as an AI server 200.

At this time, the autonomous vehicle 100b may perform an operation by generating a result using a direct learning model, but may also transmit sensor information to an external device such as an AI server 200 and perform an operation by receiving the result generated accordingly.

The autonomous vehicle 100b may determine a movement path and a driving plan using at least one of map data, object information detected from sensor information, or object information acquired from an external device, and control the driving unit to drive the autonomous vehicle 100b according to the determined movement path and driving plan.

The map data may include object identification information for various objects placed in a space (e.g., a road) where the autonomous vehicle 100b drives. For example, the map data may include object identification information for fixed objects such as streetlights, rocks, and buildings, and movable objects such as vehicles and pedestrians. In addition, the object identification information may include a name, type, distance, location, etc.

In addition, the autonomous vehicle 100b can perform an action or drive by controlling the driving unit based on the user's control/interaction. At this time, the autonomous vehicle 100b can obtain the intention information of the interaction according to the user's action or voice utterance, and determine a response based on the obtained intention information to perform the action.

### <AI+XR>

The XR device 100c can be implemented as a HMD (Head-Mounted Display), a HUD (Head-Up Display) equipped in a vehicle, a television, a mobile phone, a smart phone, a computer, a wearable device, a home appliance, a digital signage, a vehicle, a fixed robot or a mobile robot, etc., by applying AI technology.

The XR device 100c can analyze 3D point cloud data or image data acquired through various sensors or from an external device to generate location data and attribute data for 3D points, thereby acquiring information about the surrounding space or real objects, and rendering and outputting an XR object to be output. For example, the XR device 100c can output an XR object including additional information about a recognized object by corresponding it to the recognized object.

The XR device 100c can perform the above-described operations using a learning model composed of at least one artificial neural network. For example, the XR device 100c can recognize a real object from 3D point cloud data or image data using a learning model, and provide information corresponding to the recognized real object. Here, the learning model may be learned directly in the XR device 100c, or learned from an external device such as an AI server 200.

At this time, the XR device 100c may generate a result using the learning model directly and perform an operation, but may also transmit sensor information to an external device such as an AI server 200 and receive the result generated accordingly to perform an operation.

### <AI+Robot+Autonomous Driving>

The robot 100a can be implemented as a guide robot, a transport robot, a cleaning robot, a wearable robot, an entertainment robot, a pet robot, an unmanned flying robot, etc. by applying AI technology and autonomous driving technology.

The robot 100a to which AI technology and autonomous driving technology are applied can mean a robot itself with an autonomous driving function, or a robot 100a that interacts with an autonomous driving vehicle 100b, etc.

The robot 100a with an autonomous driving function can collectively refer to devices that move on their own along a given path without user control or move by determining the path on their own.

The robot 100a with an autonomous driving function and the autonomous driving vehicle 100b can use a common sensing method to determine one or more of a movement path or a driving plan. For example, a robot 100a with autonomous driving function and an autonomous vehicle 100b can determine at least one of a movement path or a driving plan by using information sensed through a lidar, a radar, and a camera.

A robot 100a interacting with an autonomous vehicle 100b can exist separately from the autonomous vehicle 100b and perform an operation linked to an autonomous driving function within the autonomous vehicle 100b or linked to a user riding in the autonomous vehicle 100b.

At this time, the robot 100a interacting with the autonomous vehicle 100b can obtain sensor information on behalf of the autonomous vehicle 100b and provide it to the autonomous vehicle 100b, or obtain sensor information and generate surrounding environment information or object information and provide it to the autonomous vehicle 100b, thereby controlling or assisting the autonomous driving function of the autonomous vehicle 100b.

Alternatively, the robot 100a interacting with the autonomous vehicle 100b may monitor a user riding in the autonomous vehicle 100b or control the functions of the autonomous vehicle 100b through interaction with the user. For example, if the robot 100a determines that the driver is drowsy, it may activate the autonomous driving function of the autonomous vehicle 100b or assist in the control of the drive unit of the autonomous vehicle 100b. Here, the functions of the autonomous vehicle 100b controlled by the robot 100a may include not only the autonomous driving function, but also functions provided by a navigation system or audio system installed inside the autonomous vehicle 100b.

Alternatively, the robot 100a interacting with the autonomous vehicle 100b may provide information to the autonomous vehicle 100b from outside the autonomous vehicle 100b or assist in the functions. For example, the robot 100a may provide traffic information including signal information, etc. to the autonomous vehicle 100b, such as a smart traffic light, or may interact with the autonomous vehicle 100b to automatically connect the electric charger to the charging port, such as an automatic electric charger for an electric vehicle.

### <AI+Robot+XR>

The robot 100a can be implemented as a guide robot, a transport robot, a cleaning robot, a wearable robot, an entertainment robot, a pet robot, an unmanned flying robot, a drone, etc. by applying AI technology and XR technology.

The robot 100a to which the XR technology is applied can mean a robot that is the target of control/interaction within an XR image. In this case, the robot 100a is distinct from the XR device 100c and can be linked with each other.

When the robot 100a that is the target of control/interaction within an XR image obtains sensor information from sensors including a camera, the robot 100a or the XR device 100c generates an XR image based on the sensor information, and the XR device 100c can output the generated XR image. And, such robot 100a can operate based on a control signal input through an XR device 100c or a user's interaction.

For example, a user can check an XR image corresponding to the viewpoint of a remotely connected robot 100a through an external device such as an XR device 100c, and through interaction, adjust the autonomous driving path of the robot 100a, control the operation or driving, or check information on surrounding objects.

### <AI+Autonomous Driving+XR>

The autonomous vehicle 100b can be implemented as a mobile robot, vehicle, unmanned aerial vehicle, etc. by applying AI technology and XR technology.

The autonomous vehicle 100b to which XR technology is applied can mean an autonomous vehicle equipped with a means for providing XR images, or an autonomous vehicle that is the subject of control/interaction within the XR images. In particular, the autonomous vehicle 100b that is the subject of control/interaction within the XR images is distinct from the XR device 100c and can be linked with each other.

The autonomous vehicle 100b equipped with a means for providing XR images can obtain sensor information from sensors including cameras and output an XR image generated based on the obtained sensor information. For example, the autonomous vehicle 100b can provide passengers with an XR object corresponding to a real object or an object on the screen by having a HUD to output an XR image.

At this time, when the XR object is output to the HUD, at least a part of the XR object can be output so as to overlap with the actual object to which the passenger's gaze is directed. On the other hand, when the XR object is output to the display equipped inside the autonomous vehicle 100b, at least a part of the XR object can be output so as to overlap with the object in the screen. For example, the autonomous vehicle 100b can output XR objects corresponding to objects such as a road, another vehicle, a traffic light, a traffic sign, a two-wheeled vehicle, a pedestrian, a building, etc.

When the autonomous vehicle 100b that is the target of control/interaction in the XR image obtains sensor information from sensors including a camera, the autonomous vehicle 100b or the XR device 100c can generate an XR image based on the sensor information, and the XR device 100c can output the generated XR image. In addition, the autonomous vehicle 100b can operate based on a control signal input through an external device such as the XR device 100c or a user's interaction.

FIG. 4 illustrates an AI device 100 according to one another embodiment of the present disclosure.

Any description overlapping with Fig. 1 is omitted.

Referring to Fig. 4, the input interface 120 may include a camera 121 to input a video signal, a micro-phone 122 to receive an audio signal, and a user input interface 123 to receive information from a user.

Voice data or image data collected by the input interface 120 may be analyzed and processed using a control command of the user.

The input interface 120, which inputs image information (or a signal), audio information (or a signal) data, or information input from a user. the AI device 100 may include one or a plurality of cameras 121 to input image information to input image information.

The camera 121 may process an image frame, such as a still image or a moving picture image, which is obtained by an image sensor in a video call mode or a photographing mode. The processed image frame may be displayed on the display 151 or stored in the memory 170.

The micro-phone 122 processes an external sound signal as electrical voice data. The processed voice data may be variously utilized based on a function (or an application program which is executed) being performed by the AI device 100. Meanwhile, various noise cancellation algorithms may be applied to the microphone 122 to remove noise caused in a process of receiving an external sound signal.

The user input interface 123 receives information from the user. When information is input through the user input interface 123, the processor 180 may control the operation of the AI device 100 to correspond to the input information.

The user input interface 123 may include a mechanical input interface (or a mechanical key, for example, a button positioned at a front/rear surface or a side surface of the terminal 100, a dome switch, a jog wheel, or a jog switch), and a touch-type input interface. For example, the touch-type input interface may include a virtual key, a soft key, or a visual key displayed on the touch screen through software processing, or a touch key disposed in a part other than the touch screen.

The output interface 150 may include at least one of a display 151, a sound output interface 152, a haptic module 153, or an optical output interface 154.

The display 151 displays (or outputs) information processed by the AI device 100. For example, the display 151 may display execution screen information of an application program driven by the AI device 100, or a User interface (UI) and graphical User Interface (GUI) information based on the execution screen information.

As the display 151 forms a mutual layer structure together with a touch sensor or is integrally formed with the touch sensor, the touch screen may be implemented. The touch screen may function as the user input interface 123 providing an input interface between the AI device 100 and the user, and may provide an output interface between a terminal 100 and the user.

The sound output interface 152 may output audio data received from the communicator 110 or stored in the memory 170 in a call signal reception mode, a call mode, a recording mode, a voice recognition mode, and a broadcast receiving mode.

The sound output interface 152 may include at least one of a receiver, a speaker, or a buzzer.

The haptic module 153 generates various tactile effects which the user may feel. A representative tactile effect generated by the haptic module 153 may be vibration.

The light outputting interface 154 outputs a signal for notifying that an event occurs, by using light from a light source of the AI device 100. Events occurring in the AI device 100 may include message reception, call signal reception, a missed call, an alarm, schedule notification, email reception, and reception of information through an application.

Meanwhile, the communicator 110 may also be named a communication interface 110.

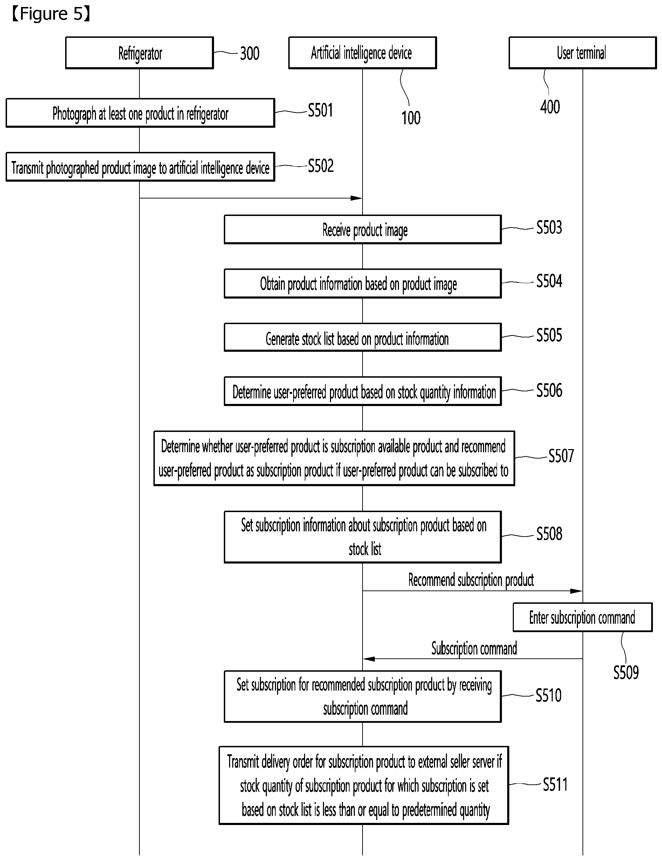

FIG. 5 is a flowchart for explaining a product recommendation method according to an embodiment of the present disclosure.

The refrigerator 300 can photograph at least one product in the refrigerator (S501).

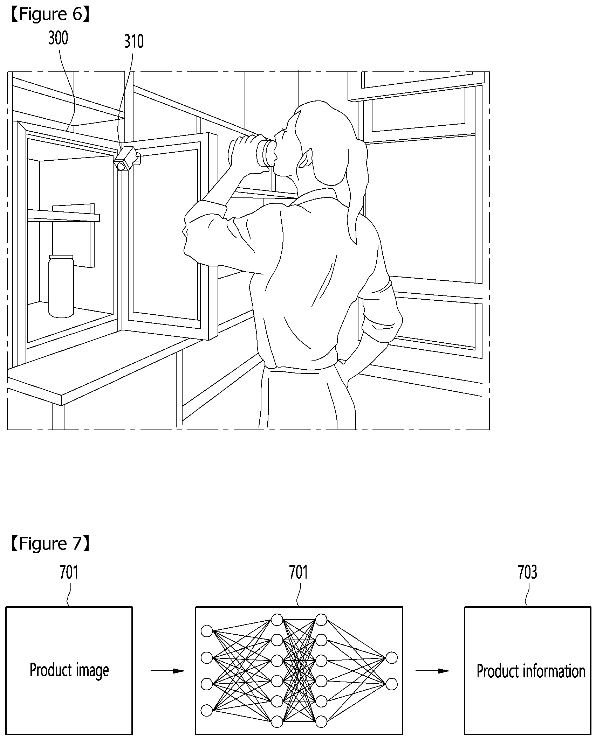

FIG. 6 is a drawing for explaining a refrigerator that photographs a product according to an embodiment of the present disclosure.

Referring to FIG. 6, the refrigerator 300 can include a camera 310 that photographs a product stored in at least one separate storage tube in the refrigerator.

The camera 310 can photograph at least one product in the refrigerator when the refrigerator is opened and then closed.

Meanwhile, the refrigerator 300 can transmit the photographed product image to the artificial intelligence device 100 (S502).

For example, the refrigerator 300 can transmit a product image of at least one product stored in the refrigerator to the artificial intelligence device 100 every time the refrigerator is opened and closed. Therefore, the refrigerator 300 can capture a product image of a product stored in the refrigerator and transmit it to the artificial intelligence device 100 every time the user puts a product in or takes it out of the refrigerator.

Meanwhile, the artificial intelligence device 100 can receive a product image transmitted from the refrigerator 300 (S503). The communicator 110 of the artificial intelligence device 100 can receive the product image transmitted from the refrigerator 300. The product image may be an image in which at least one product is captured in a refrigerator.

Meanwhile, the artificial intelligence device 100 can obtain product information based on the received product image (S504). The processor 180 of the artificial intelligence device 100 can obtain product information based on the received product image. The processor 180 can obtain at least one product information based on a product image in which at least one product is photographed.

The product information can include at least one of category information, product name information, and nutritional information about the product. For example, the category information is information for product differentiation, such as bottled water, carbonated water, beer, soju, juice, carbonated beverage, coke, cider, milk, yogurt, ketchup, and fruit. In addition, the product name information can include information such as the brand name and manufacturing company name of the product, and can include information about the fruit name if the product belongs to the fruit category. In addition, the nutritional information can be information related to nutritional information of the product, such as carbohydrates, dietary fiber, and sugar.

Meanwhile, the processor 180 can input a product image into a product recognition model and obtain product information output by the product recognition model.

FIG. 7 is a drawing for explaining a product recognition model according to an embodiment of the present disclosure.

Referring to FIG. 7, the processor 180 can input a product image 701 into a product recognition model 702 and obtain product information 703 output by the product recognition model 702.

The product recognition model may be an artificial neural network model trained to output predetermined product information from a predetermined product image.

In addition, the product recognition model may be an artificial neural network model trained to output predetermined product information from a predetermined product image.

The product recognition model may be an artificial neural network (ANN) model used in machine learning. The product recognition model may be composed of artificial neurons (nodes) that form a network by combining synapses. A product recognition model can be defined by the connection patterns between neurons in different layers, the learning process that updates model parameters, and the activation function that generates the output values.

The product recognition model may include an input layer, an output layer, and optionally one or more hidden layers. Each layer may include one or more neurons, and the artificial neural network may include neurons and synapses connecting neurons. In the artificial neural network, each neuron may output a function value of an activation function for input signals, weights, and biases input through the synapses.

The product recognition model may be created through Supervised Learning, Unsupervised Learning, or Reinforcement Learning depending on the learning method.

For example, if the product recognition model is created through supervised learning, it may be learned in a state a label for the learning data is given. The label may mean the correct answer (or result value) that the artificial neural network must infer when the learning data is input to the artificial neural network.

The learning processor 130 can designate a label that specifies product information for a given product image. For example, category information and product name information can be designated for each of various product images.

Therefore, the learning processor 130 can train a product recognition model to output product information by labeling product information corresponding to the input product image when the product image is input.

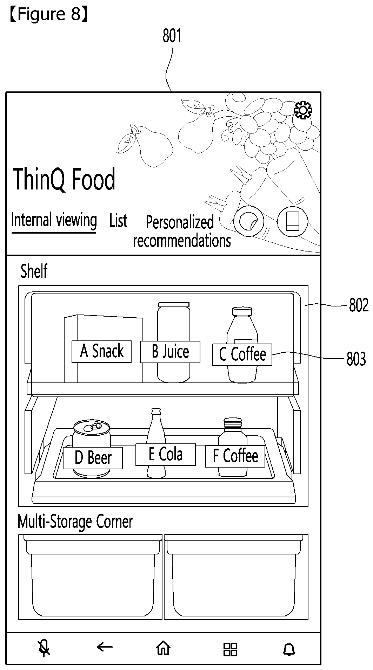

Meanwhile, FIG. 8 is a drawing for explaining a product image in which product information is recognized according to one embodiment of the present disclosure.

Referring to FIG. 8, the communicator 110 of the artificial intelligence device 100 can transmit the product image 802 and product information 803 recognized from the product image 802 to the user terminal 400, and the user terminal 400 can display the received product image 802 and product information 803 through the refrigerator internal viewing interface 801.

In this case, the user terminal 400 can be implemented as a fixed device or a movable device such as a TV, a mobile phone, a smart phone, a desktop computer, a laptop, a tablet PC, a wearable device, etc., as a device that can be linked with the refrigerator 300 and the artificial intelligence device 100.

Meanwhile, the processor 180 of the artificial intelligence device 100 can generate or update a stock list based on the acquired product information (S505).

The stock list may include product information and stock quantity information for each of at least one product stored in the refrigerator.

The processor 180 can generate a stock list for products stored in the refrigerator based on at least one piece of product information. In addition, if a stock list has already been generated, the processor 180 can update newly added products and added quantities, withdrawn products and withdrawn quantities, etc. based on the product information.

In addition, the processor 180 can store a stock list including product information and stock quantity information for each product based on at least one piece of acquired product information in the memory 170.

Meanwhile, the processor 180 can determine a user-preferred product based on the stock quantity information for each of at least one product (S505).

The processor 180 can determine a product that maintains a predetermined stock quantity for a predetermined period of time as a product preferred by the user based on the stock quantity information of at least one product included in the stock list.

For example, the processor 180 may determine product 'A' as the user's preferred product if the stock quantity of product 'A' is maintained at least 1 for more than 2 months.

Meanwhile, the processor 180 can determine whether the user-preferred product is a product that can be subscribed to, and if it is a product that can be subscribed to, can recommend it as a subscription product (S507).

A subscription product may mean a product that a product seller can regularly deliver to a designated address.

The memory 170 can store subscription product information that can be regularly delivered by each of at least one product supplier or seller.

For example, the processor 180 may receive a list of subscription available products from at least one external seller server (not shown) through the communicator 110 and store the list in the memory 170.

The processor 180 may compare the user-preferred product with the list of subscription available products to determine whether the user-preferred product is a subscription available product.

If the user-preferred product is a subscription available product, the processor 180 may recommend it as a subscription product (S507). The processor 180 may transmit information about the recommended subscription product to the user terminal 400 through the communicator 110.

In addition, if the processor 180 recommends a subscription product, it may set subscription information about the recommended subscription product (S508).

The subscription information may include information about the subscription period and subscription quantity for the subscription product. For example, the subscription information may include information about the subscription period, '1 month', and the subscription quantity o, '5', for the recommended subscription product 'A'.

The processor 180 may set the subscription information including the information about the subscription period and subscription quantity for the recommended subscription product as the subscription information for the recommended subscription product.

Meanwhile, the processor 180 may set the subscription information for the subscription product based on the stock list when recommending the subscription product.

The processor 180 may set the subscription information for the recommended subscription product based on the stock quantity for a predetermined period of time for the recommended subscription product based on the stock list.

For example, the processor 180 can obtain the average stock quantity (5) for a predetermined subscription period (e.g., 1 month) of the recommended subscription product based on the stock list, and set the obtained average stock quantity as the subscription quantity.

The processor 180 can transmit information on the recommended subscription product and the set subscription information to the user terminal 400 through the communicator 110.

The user terminal 400 can display the received recommended subscription product information and subscription information, and receive a command regarding whether to subscribe from the user (S509). When the user terminal 400 receives a subscription command, the user terminal 400 can transmit the received subscription command to the artificial intelligence device 100.

Meanwhile, the processor 180 can receive a subscription command from the user terminal 400 through the communicator 110 and set a subscription for the recommended subscription product (S510).

If the stock quantity of a subscription product for which a subscription is set based on a stock list is less than a predetermined quantity, the processor 180 can transmit a delivery order for the subscription product to an external seller server (not shown) through the communicator 110 (S511). Accordingly, the stock quantity of a product subscribed by a user among products stored in a refrigerator can be maintained at a constant level, thereby promoting user convenience.

Meanwhile, if the processor 180 transmits a delivery order for a subscription product to an external seller server (not shown), it can transmit an additional purchase alarm to the user terminal 400.

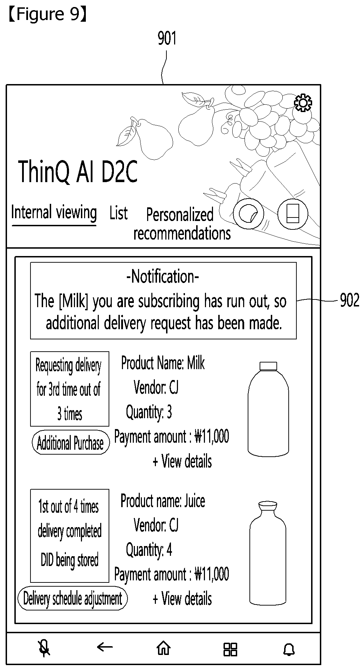

FIG. 9 is a drawing showing a delivery order alarm screen according to one embodiment of the present disclosure.

Referring to FIG. 9, the user terminal 400 can output a purchase notification interface 901 including an additional purchase notification message 902 for the subscription product.

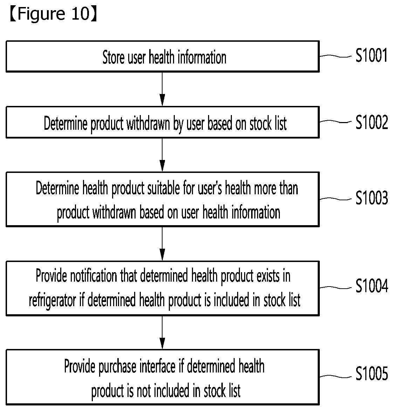

Meanwhile, FIG. 10 is a flowchart for explaining a product recommendation method based on user health information according to one embodiment of the present disclosure.

The processor 180 can receive user health information from the user terminal 400 through the communicator 110 and store the user health information in the memory 170 (S1001).

The user health information may include medical history information as information related to the user's health status. For example, the medical history information may include disease information such as diabetes, hypertension, and arrhythmia.

The processor 180 can determine the product withdrawn by the user based on the stock list (S1002).

For example, when the user opens the door of the refrigerator 300, takes out the first product, and closes the door, the camera 310 of the refrigerator 300 can capture at least one product in the refrigerator 300 and transmit the captured product image to the artificial intelligence device 100. In addition, the processor 180 can obtain product information based on the received product image and update the stock list based on the obtained product information. Additionally, the processor 180 can determine a product with a reduced stock quantity as the first product withdrawn from the refrigerator 300 based on the updated stock list.

Meanwhile, the processor 180 can determine a health product suitable for the user's health more than the product withdrawn based on the user health information (S1003).

For example, the processor 180 can compare the user's health information with the nutritional content information included in the product information of the extracted product to determine nutritional content that has a negative effect on the user's health, and determine another product that contains less nutritional content with a negative effect than the extracted product as a healthy product.

For example, if the medical history information included in the user health information is diabetes and the extracted product contains a sugar component that has a negative effect on the user's health, the processor 1800 can determine another product that contains less sugar than the extracted product as a health product.

Meanwhile, if the determined health product is included in the stock list, the processor 180 can provide a notification that the determined health product exists in the refrigerator (S1004).

For example, if the determined health product is included in the stock list, the processor 180 can transmit product information about the determined health product and a notification that the determined health product exists in the refrigerator to the user terminal 400 through the communicator 110.

In addition, if the determined health product is not included in the stock list, the processor 180 can provide a purchase interface (S1005).

For example, the processor 180 may transmit a purchase interface including a site link for purchasing the determined health product to the user terminal 400.

The above-described present disclosure can be implemented as a computer-readable code on a medium in which a program is recorded. The computer-readable medium includes all types of recording devices in which data that can be read by a computer system is stored. Examples of the computer-readable medium include a hard disk drive (HDD), a solid state disk (SSD), a silicon disk drive (SDD), a ROM, a RAM, a CD-ROM, a magnetic tape, a floppy disk, an optical data storage device, and the like. In addition, the computer may include a processor 180 of an artificial intelligence device.

## Claims

1. An artificial intelligence device, comprising:
a communicator configured to receive a product image in which at least one product is captured in a refrigerator; and
a processor configured to obtain at least one product information based on the product image, generate a stock list for products stored in the refrigerator based on the product information, determine a user-preferred product based on a change in the stock quantity of each of at least one product included in the stock list, and determine whether the user-preferred product is a subscription available product and recommend the user-preferred product as a subscription product if the user-preferred product is determined as subscription available product.

2. The artificial intelligence device of claim 1, further comprising a memory configured to store a list of subscription available products, and
wherein the processor is configured to compare the user-preferred product and the list of subscription available products to determine whether the user-preferred product is the subscription available product.

3. The artificial intelligence device of claim 1, wherein the processor is configured to set subscription information for the recommended subscription product, and
the subscription information includes information about a subscription period and subscription quantity for the recommended subscription product.

4. The artificial intelligence device of claim 3, wherein the processor is configured to set subscription information for the recommended subscription product based on a stock quantity of the recommended subscription product for a predetermined period of time based on the stock list.

5. The artificial intelligence device of claim 4, wherein the processor is configured to obtain an average stock quantity for the recommended subscription product for a predetermined subscription period based on the stock list, and set the obtained average stock quantity as a subscription quantity.

6. The artificial intelligence device of claim 3, wherein the processor is configured to transmit information on a recommended subscription product and set subscription information to a user terminal through the communicator, and receive a subscription command from the user terminal through the communicator to set a subscription to the recommended subscription product.

7. The artificial intelligence device of claim 6, wherein the processor is configured to transmit a delivery order for the subscription product for which the subscription is set to an external seller server through the communicator if a quantity of the subscription product for which the subscription is set is less than or equal to a predetermined number based on the stock list.

8. The artificial intelligence device of claim 7, wherein the processor is configured to transmit an additional purchase alarm to the user terminal.

9. The artificial intelligence device of claim 1, further comprising a memory configured to store user health information, and
wherein the processor is configured to determine a product withdrawn by the user based on the stock list, and determine a health product suitable for the user's health more than the withdrawn product based on the user health information.

10. The artificial intelligence device of claim 9, wherein the processor is configured to determine nutritional content that has a negative effect on a user's health by comparing the user health information with nutritional content information included in a product information of the withdrawn product, and determine another product that contains less nutritional content that has a negative effect than the withdrawn product as the health product.

11. The artificial intelligence device of claim 9, wherein the processor is configured to provide a notification that the determined health product exists in the refrigerator if the determined health product is included in the stock list.

12. The artificial intelligence device of claim 9, wherein the processor is configured to provide a purchasing interface if the determined health product is not included in the stock list.

13. A method of recommending product, comprising:
receiving a product image in which at least one product is captured in a refrigerator;
obtaining at least one product information based on the product image;
generating a stock list for products stored in the refrigerator based on the product information;
determining a user-preferred product based on a change in stock quantity of each of at least one product included in the stock list; and
determining whether the user-preferred product is a subscription available product and recommending the user-preferred product as a subscription product if the user-preferred product is determined a subscription available product.

14. The method of recommending product of claim 13, further comprising:
determining a product withdrawn by a user based on the stock list;
determining a health product suitable for the user's health more than the product withdrawn based on the user health information.
